# EUROPEAN PATENT APPLICATION

(11) **EP 4 589 009 A1**
(43) Date of publication of application: **23.07.2025**
(21) Application number: 24775027.6
(22) Date of filing: 03.01.2024
(51) Int. Cl.: C12P 13/04, C12N 15/01, C12N 1/20, C12R 1/15

(54) **MICROORGANISMS WITH ENHANCED GLYCINE PRODUCTION CAPABILITY AND METHOD FOR PRODUCING GLYCINE USING SAME**

(30) Priority: 22.03.2023 KR 20230037523
(71) Applicant: CJ CheilJedang Corporation, Seoul 04560 (KR)
(72) Inventor: JUNG, Hwi-Min, Seoul 04560 (KR); RHO, Jin Ah, Seoul 04560 (KR); CHO, Hyunjin, Seoul 04560 (KR); CHOI, Jin-Geun, Seoul 04560 (KR)
(74) Representative: Meissner Bolte Partnerschaft mbB
(86) International application number: PCT/KR2024/000087
(87) International publication number: WO 2024/195979

(57) **Abstract**

The present invention relates to a Corynebacterium sp. mutant strain, a method for producing the strain, and a method for producing glycine using the strain, wherein the strain is induced to mutate by radiation exposure to have enhanced glycine production capability compared to the parent strain. The Corynebacterium mutant strain exhibits significantly improved glycine production capability compared to the parent strain, enabling high-efficiency and high-yield glycine production.

## Description

### [TECHNICAL FIELD]

### Cross-reference to related application(s)

The present application claims the benefit of the priority based on Korean patent application No. 10-2023-0037523 on March 22, 2023, and the entire contents disclosed in the documents of the corresponding patent application are incorporated as a part of the present description.

The present invention relates to a microorganism with improved glycine productivity and a method for producing glycine using the same.

### [BACKGROUND ART]

Glycine is an important amino acid that is widely applied in fields such as chemistry, food, medicine and the like. Glycine is an amino acid making a mild sweet taste, and in particular, it has been used in the food industry as a flavoring agent and a nutritional supplement. Based on 2020, the global glycine market is evaluated at 700 million USD or more, and is expected to show a growth rate of about 4% annually.

In general, glycine is obtained by chemical synthesis or protein hydrolysis. Chemical chemistry is achieved through reaction of chloroacetic acid, trichloroethylene, tetrachloroethane and ammonia, or Strecker synthesis, and protein hydrolysis is not often used industrially. Most industrial production has problems in that it is accomplished through chemical synthesis, and a high temperature/high pressure process is necessary, and a substrate with toxicity to human bodies is used.

Glycine fermentation by microorganisms is conducted under conditions of room temperature and room pressure, and suggests a process of utilizing a safe carbon source. Naturally, microorganisms form glycine using an L-serine biosynthesis pathway or L-threonine degradation pathway. However, there are limitations to producing glycine using a naturally derived strain due to low production concentration.

Accordingly, efforts to develop a microorganism producing glycine with high efficiency or a fermentation process technology are being made. Specifically, a method for strengthening activity of an enzyme involved in an amino acid synthesis pathway in a *Corynebacterium* sp. microorganism (US 11661616 B2) and the like have been used. However, a need of research on a method capable of producing glycine efficiently and in high yield are still being raised.

Under this background, the present inventors have made diligent efforts to develop a microorganism producing glycine having a higher producing effect than conventional strains, and as a result, have discovered a fact that a specific *Corynebacterium* mutant strain produces glycine at high yield, thereby completing the present invention.

### [DISCLOSURE]

### [TECHNICAL PROBLEM]

One embodiment of the present invention provides a *Corynebacterium* sp. mutant strain with improved glycine productivity compared to a parent strain, which is a mutant strain obtained by mutating a parent strain, *Corynebacterium glutamicum* ATCC13032 strain.

Another embodiment of the present invention provides a method for preparing of a mutant strain with improved glycine productivity compared to a parent strain, comprising
1) irradiating radioactive rays to a parent strain, *Corynebacterium glutamicum* ATCC13032 to mutate it; and
2) culturing the mutated strain of the step 1) in a medium to select a mutant strain with improved glycine productivity.

Other embodiment of the present invention provides a composition for producing glycine comprising the microorganism with improved glycine productivity.

Other embodiment of the present invention provides a method for producing glycine, comprising culturing the microorganism with improved glycine productivity in a medium.

Other embodiment of the present invention provides a use of the microorganism with improved glycine productivity for using in glycine production.

### [TECHNICAL SOLUTION]

This is explained in detail as follows. On the other hand, each description and embodiment disclosed in the present application can also be applied to each other description and embodiment. In other words, all combinations of the various elements disclosed in the present application belong to the scope of the present application. In addition, the scope of the present application cannot be considered limited by the specific description described below. Furthermore, those skilled in the art can recognize or confirm many equivalents to the specific embodiments of the present application described in the present application using only a common experiment. In addition, these equivalents are intended to be incorporated in the present application.

The present invention provides a microorganism with improved glycine productivity.

In the present invention, screening to discover a mutant strain highly producing glycine was performed. As a result, it was confirmed that 3 kinds of the mutant strains prepared by irradiating radioactive rays to the *Corynebacterium glutamicum* ATCC13032 strain had improved glycine productivity compared to the ATCC13032 strain.

The present invention provides a *Corynebacterium* sp. mutant strain with improved glycine productivity compared to a parent strain, which is a mutant strain obtained by mutating a parent strain, *Corynebacterium glutamicum* ATCC13032.

The microorganism may be a *Corynebacterium glutamicum* mutant strain.

The *Corynebacterium glutamicum* mutant strain may be any one strain selected from the group consisting of *Corynebacterium glutamicum* CJ2004 strain (Accession number KCCM13317P), *Corynebacterium glutamicum* CJ2029 strain (Accession number KCCM13318P) and *Corynebacterium glutamicum* CJ2049 strain (Accession number KCCM13319P).

In the present description, the term, "glycine" is an amino acid of a colorless crystal making a sweet taste, and is named glycine, and refers to an amino acid with a chemical formula "C₂H₅NO₂".

In the present description, the term, "productivity" means an ability capable of producing glycine, and may be interchangeably used with "production", and can be confirmed by measuring the concentration of glycine comprised in the cultured solution after culturing a strain producing glycine.

The mutation method may be performed by various means known in the corresponding field, and one method of physical or chemical mutagenesis methods may be used. For example, as a physical mutation method suitable for the present invention, a method for irradiating gamma rays or ultraviolet rays may be used, but is not limited thereto. In addition, as a chemical mutation method, N-methyl-N'-nitro-N-nitrosoguanidine (NTG), diepoxy butane, ethyl methane sulfonate, mustard compounds, hydrazine and nitrite may be used, but are not limited thereto.

Mutation for preparing the mutant strain may be by a method for irradiating radioactive rays.

Radioactive rays to cause the mutation may be gamma rays or ultraviolet rays, and preferably, gamma rays.

The radioactive rays may have a dose of 3.0 kGy to 5.0 kGy, 3.1 kGy to 4.9 kGy, 3.2 kGy to 4.8 kGy, 3.3 kGy to 4.7 kGy, 3.4 kGy to 4.6 kGy, 3.5 kGy to 4.5 kGy, 3.6 kGy to 4.4 kGy, 3.7 kGy to 4.3 kGy, 3.8 kGy to 4.2 kGy, 3.9 kGy to 4.1 kGy, preferably, 4.0 kGy, per hour, but are not limited thereto.

The mutant strain may have increased glycine productivity by 20% or more, and may have that increased by 22% or more, and may have that increased by 25% or more, and may have that increased by 30% or more, and may have that increased by 40% or more, and may have that increased by 50% or more, and may have that increased by 60% or more, and may have that increased by 70% or more, compared to the parent strain.

The parent strain may be *Corynebacterium glutamicum* ATCC13032, but is not limited thereto.

In addition, the present invention provides a method for preparing of a mutant strain with improved glycine productivity compared to a parent strain, comprising
1) irradiating radioactive rays to a parent strain, *Corynebacterium glutamicum* ATCC13032 to mutate it; and
2) culturing the mutated strain of the step 1) in a medium to select a mutant strain with improved glycine productivity.

Radioactive rays to cause the mutation may be gamma rays or ultraviolet rays, and preferably, gamma rays.

The radioactive rays may have a dose of 3.0 kGy to 5.0 kGy, 3.1 kGy to 4.9 kGy, 3.2 kGy to 4.8 kGy, 3.3 kGy to 4.7 kGy, 3.4 kGy to 4.6 kGy, 3.5 kGy to 4.5 kGy, 3.6 kGy to 4.4 kGy, 3.7 kGy to 4.3 kGy, 3.8 kGy to 4.2 kGy, 3.9 kGy to 4.1 kGy, preferably, 4.0 kGy, per hour, but are not limited thereto.

During mutagenesis, the parent strain is affected by a mutagenic factor under conditions that leave viable organisms in a certain size. The conditions vary depending on the type of the mutagenic factors, and depend on the amount of mutations that the mutagenic factors cause in the viable organisms at a certain death rate (killing rate). For example, in case of gamma rays, the death rate may leave approximately 0.00001% to 20% of the starting population, and in case of NTG, the killing rate may leave approximately 10% to 50% of the starting population, and the occurrence of mutations by nitrite may leave 0.01% to 0.1% of the starting population, but is not limited thereto.

In addition, the present invention provides a composition for producing glycine comprising a *Corynebacterium* sp. mutant strain with improved glycine productivity compared to a parent strain, which is a mutant strain obtained by mutating a parent strain, *Corynebacterium glutamicum* ATCC13032.

The microorganism may be a *Corynebacterium glutamicum* mutant strain.

The *Corynebacterium glutamicum* mutant strain may be any one strain selected from the group consisting of *Corynebacterium glutamicum* CJ2004 strain (Accession number KCCM13317P), *Corynebacterium glutamicum* CJ2029 strain (Accession number KCCM13318P) and *Corynebacterium glutamicum* CJ2049 strain (Accession number KCCM13319P).

In one example of the present invention, it was confirmed that the *Corynebacterium glutamicum* CJ2004 strain (Accession number KCCM13317P), *Corynebacterium glutamicum* CJ2029 strain (Accession number KCCM13318P) and *Corynebacterium glutamicum* CJ2049 strain (Accession number KCCM13319P), which were obtained by mutating a parent strain, *Corynebacterium glutamicum* ATCC13032, produced glycine at a high concentration. Therefore, a composition comprising the mutant strain can produce glycine at a high concentration.

Furthermore, the present invention provides a method for producing glycine comprising culturing a *Corynebacterium* sp. mutant strain with improved glycine productivity compared to a parent strain, which is a mutant strain obtained by mutating a parent strain, *Corynebacterium glutamicum* ATCC13032.

The method may further comprise recovering glycine from the cultured microorganism, culture products, or both of them, after the culturing.

The term of the present description, "culture" means growing a microorganism under a properly artificially controlled environmental condition. The method for culturing a microorganism of the present invention may be performed using a culturing method of *Corynebacterium glutamicum* widely known in the art. Specifically, the example of the culturing method includes batch culture, continuous culture, and fed-batch culture, but is not limited thereto. These various methods are disclosed, for example, "Biochemical Engineering"(James M. Lee, Prentice-Hall International Editions, pp138-176, 1991), and the like.

The term of the present description, "culture products" mean substances comprising a medium in which a microorganism is growing or has grown under a properly artificially controlled environmental condition. In a narrow meaning, the culture products may include grown microorganisms, but in a broad meaning, it may be included. The "culture products" include various substances excreted with the medium components into the medium created for culturing microorganisms by microorganisms during growing, and specifically, a target substance, glycine is included.

The media used for culture should satisfy requirements of a specific strain in an appropriate way. The culture media for the *Corynebacterium* sp. strain are known. For example, the microorganism of the present application may be cultured while adjusting the temperature, pH and the like under an aerobic condition in a common medium containing proper carbon sources, nitrogen sources, amino acids, vitamins, and the like. Then, as the carbon sources, carbohydrates such as glucose, fructose, and sucrose, amino acids such as glutamic acid and cysteine, and the like are included. Specifically, natural organic nutrients such as starch hydrolysate and molasses may be used, and preferably, they may be carbohydrates such as glucose, fructose, sterilized pretreated molasses (that is, molasses converted with reducing sugar), and the like, and other carbon sources in an appropriate amount may be variously used without limitations, but are not limited thereto. As the nitrogen sources, inorganic nitrogen sources such as ammonia; and organic nitrogen sources such as amino acids such as glutamic acid and cysteine and peptone, meat extract, yeast extract and the like may be used. These nitrogen sources may be used alone or in combination, but are not limited thereto. In the medium, as the phosphorus sources, phosphate, potassium dihydrogen phosphate or dipotassium hydrogen phosphate, or corresponding nitrogen-containing salts may be used, but are not limited thereto. As an inorganic compound, magnesium sulfate, iron sulfate, manganese sulfate and calcium chloride and the like may be used, and in addition, amino acids, vitamins and appropriate precursors and the like may be comprised. These media or precursors may be added to culture products in a batch or continuous manner, but are not limited thereto.

During culturing, by adding a compound such as potassium hydroxide, ammonia and phosphate by an appropriate method, the pH of culture products may be adjusted. In addition, during culturing, using an antifoaming agent such as fatty acid polyglycol ester, bubble generation may be inhibited. Furthermore, in order to maintain an aerobic state of the culture products, oxygen or oxygen-containing gas may be injected in the culture products. The temperature of the culture products may be 27°C to 37°C, specifically, 30°C to 33°C. The culturing period may continue until the produced amount of desired useful substances is obtained, and specifically, it may be 20 to 120 hours.

The recovering glycine may collect targeted glycine from the medium, cultured solution, or microorganism using a proper method known in the art according to the culturing method. For example, the recovering may be performed by at least one method selected from centrifugation, filtration, anion exchange chromatography, crystallization, HPLC and the like, but is not limited thereto. The method for producing glycine, may additionally comprise a purifying, before, at the same time as, or after the recovering.

### [ADVANTAGEOUS EFFECTS]

The *Corynebacterium* sp. mutant strain of the present invention is a microorganism with improved glycine productivity by mutagenesis compared to a parent strain. Accordingly, by the method for producing glycine of the present invention using the *Corynebacterium* sp. mutant strain, glycine can be produced with high efficiency and high yield.

### [MODE FOR INVENTION]

Hereinafter, the present invention will be described in more detail by the following examples. However, these examples are intended to illustrate the present invention only, but the scope of the present invention is not limited by these examples.

### Example 1. Selection of mutant strains through artificial mutation method

### 1-1. Establishment of conditions of irradiating gamma rays to obtain mutant strains with improved glycine productivity

In order to obtain microbial mutant strains with improved glycine productivity, microbial mutation was induced using the following method.

To induce mutation, a physical method, a gamma ray irradiation method was applied. High-energy gamma rays emitted from a ray source, Co-60, flow along chromosomes and randomly trigger changes in base sequences such as base substitution, deletion, insertion and the like. The level of the changes in base sequences is proportional to the intensity of irradiation of gamma rays, so high-level gamma rays not only cause mutations at a high ratio, but also increase the death rate (killing rate) of the strain. In order to sufficiently secure a high-quality mutant library with high diversity at a high mutation ratio, establishment of conditions of irradiating gamma rays for the *Corynebacterium* strain, subject to irradiation of gamma rays, was preceded.

Using a high-level gamma ray irradiating device of Nordion company located at Korean Atomic Energy Research Institute, Advanced Radiation Technology Institute, gamma rays with an intensity of 0, 0.5, 1, 2, 3, 4, 5, 7.5, 10 kGy/hr, respectively, to 30 mL of seed medium cultured solution with an absorbance 562 nm value of 6.09 were irradiated for 1 hour, and then the gamma ray-irradiated solution diluted at a dilution ratio of 10⁰, 10⁻¹, 10⁻², 10⁻³, 10⁻⁴ was smeared on an activation medium by 100 µℓ, respectively, and a plate medium in which the gamma ray-irradiated solution was smeared was cultured in a 30°C stationery incubator for 48 hours, and then the number of individual colonies produced was counted to calculate the death rate (killing rate) by each irradiation condition. CFU/mL according to the gamma ray irradiation condition 0, 0.5, 1, 2, 3, 4, 5, 7.5 10 kGy/hr was 3.6 X 10⁸, 6.9 X 10⁷, 9.8 X 10⁶, 2.7 X 10⁵, 2.1 X 10³, 3.0 X 10¹, 0, 0, 0, respectively, and the death rate calculated therefrom was shown as 0%, 81.00467%, 97.31540%, 99.92589%, 99.99942%, 99.99999%, 100%, 100%, 100%, respectively. Considering that the colonies could not be secured as all were killed under the gamma ray irradiation condition with an intensity of 5 kGy/hr, an intensity of 4 kGy/hr was established as the gamma ray irradiation condition to secure a high-quality mutant library with high mutation diversity through Corynebacterium strain-based gamma ray irradiation.

### 1-2. Selection of mutant strains with improved glycine productivity

In order to obtain microorganisms with glycine high-productivity, for the *Corynebacterium glutamicum* ATCC13032 strain, a gamma ray irradiation-based mutant library was constructed, and glycine high-productivity mutants were screened. For irradiation of gamma rays for the ATCC13032 strain, it was cultured in a shaking incubator of 30°C in a 500 mL flask comprising a seed medium of 50 mL for 24 hours, and a cultured solution with an absorbance 562 nm value of 7.84 was secured, and then it was diluted so that the absorbance 562 nm value was 6.00 using the seed medium. Using a high-level gamma ray irradiating device located at Korean Atomic Energy Research Institute, Advanced Radiation Technology Institute, gamma rays of 4 kGy/hr was irradiated to the diluted solution 30 mL for 1 hour, and then the crude solution of the irradiated solution was divided and smeared on the activation medium of 100 sheets of 90 X 15 mm petri dishes, by 100 *µ*ℓ, respectively. Then, the petri dishes were cultured in a 30°C stationery incubator for 48 hours to secure a total of about 40,000 individual colonies, and for securing stability of the mutants, all the individual colonies produced in the whole petri dishes were recovered, and suspended in a 20% glycerol solution, and then subdivided by 1 mL in a 1.5 mL microtube, and stored in a ultralow freezer of -80°C.

When the glycine high-productivity mutant strains were screened, 20% glycerol suspension taken out of the ultralow freezer and thawed at a room temperature was continuously diluted to 10⁻¹, 10⁻², 10⁻³, 10⁻⁴, 10⁻⁵ using saline, and then the diluted solution corresponding to each dilution factor was smeared on the activation medium by 100 *µ*ℓ, and through culturing in a 30°C stationery incubator for 24 hours, they were reactivated in a form of individual colonies. The reactivated individual colonies were inoculated into a 96 Deep Well Plate, in which the production medium 350 *µ*ℓ corresponding to the filling rate 17% per each well, was aliquoted using Qpix420 equipment, Colony Picker of Molecular Devices company, and The ATCC13032 strain in which gamma rays were not irradiated was inoculated in 4 wells per plate on the same plate, and used as a control group for screening high-productivity mutants.

The 96 Deep Well Plate, in which the control group strain and mutant were inoculated, respectively, was sealed using Gas permeable seal mark2 of Azenta company, and then cultured under conditions of 30°C, 1,000 rpm in Multitron equipment, an Infors-HT company's shaking incubator, for 48 hours. The 96 Deep Well Plate cultured for 48 hours was under centrifugation in Centrifuge 5810R equipment, an Eppendorf company's centrifuge, under conditions of 15°C, 4,000 rpm for 20 minutes, and then for NIR Spectrometry analysis, using Biomek i5, a Beckman Coulter company's Liquid handler equipment, the cultured supernatant 100 *µ*ℓ in which microbial cells were isolated was moved to 96 Well Black Polystyrene Microplate of Corning company. After that, individual analysis data by each well were obtained by applying to NIR Spectrometry self-produced in CJ CheilJedang BIO Technology Research Institute Analysis&Quality Department, and a selection logic of improvement of the glycine concentration by 20% or more compared to the control group was applied, and 48 strains were primarily selected among 10032 mutant strains. For the selected 48 kinds, culturing was conducted by the same method as above, and top 3 kinds of strains with high glycine concentration were finally selected.

The mutant strains obtained by the method were named *Corynebacterium glutamicum* CJ2004, CJ2029, and CJ2049, and deposited to an international depository authority under Budapest Treaty, Korean Culture Center of Microorganisms on January 6, 2023 and given accession numbers KCCM13317P, KCCM13318P and KCCM13319P, respectively.

The compositions of the media used in Example 1 and Example 2 are as follows.

### <Activation medium>

Beef extract 5 g/L, Polypeptone 10 g/L, Yeast extract 5 g/L, Urea 2 g/L, sodium chloride (NaCl) 2.5 g/L, Agar 20 g/L, Glucose 10 g/L, 10N sodium hydroxide (NaOH) 100 *µ*ℓ/L

### <Seed medium>

Glucose (anhydrous glucose) 20 g/L, Polypeptone 10 g/L, Yeast extract 10 g/L, ammonium sulfate [(NH₄)₂SO₄] 10 g/L, Urea 1.5 g/L, potassium phosphate monobasic (KH₂PO₄) 5.2 g/L, potassium phosphate dibasic (K₂HPO₄) 10.7 g/L, d-Biotin 1.8 mg/L, Thiamine-HCl 9 mg/L, CAPA 9 mg/L, NCA 60 mg/L, magnesium sulfate (MgSO₄) 0.5 g/L

### <Production medium>

Calcium carbonate (CaCO₃) 30 g/L, Sucrose 50 g/L, MgSO₄ 0.6 g/L, (NH₄)₂SO₄ 20 g/L, KH₂PO₄ 1 g/L, Yeast extract 5 g/L, d-Biotin 0.05 mg/L, Thiamine-HCl 0.1 mg/L, MnSO₄ 18 µg/L, FeSO₄ 18 µg/L, ZnSO₄ 0.9 µg/L, CuSO₄ 0.9 µg/L

### Example 2. Evaluation of glycine productivity of glycine producing mutant strains

In order to confirm the glycine productivity of the *Corynebacterium glutamicum* CJ2004, CJ2029 and CJ2049 strains obtained in Example 1 above, they were cultured by the following method.

Specifically, after inoculating the *Corynebacterium glutamicum* ATCC13032 strain, which is a parent strain, and the 3 kinds of the mutant strains into a 250 ml corner-baffled flask containing a seed medium of 25 ml, respectively, they were cultured with shaking at 200 rpm at 30°C for 20 hours to obtain a seed cultured solution. After that, the seed cultured solution of 1 ml was inoculated to a 250 ml corner-baffled flask containing the production medium of 24 ml, and they were cultured at 200 rpm at 30°C for 72 hours to produce glycine. After completing the culturing, the concentration of glycine comprised in the cultured solution was measured using high-performance liquid chromatography (HPLC) to measure the produced amount of glycine of each strain. The obtained result was shown in Table 1 below.

**[Table 1]**

| Comparison of glycine productivity of *Corynebacterium glutamicum* CJ2004 strain, CJ2029 strain and CJ2049 strain | | | | |
|---|---|---|---|---|
| Strain | ATCC 13032 (Parent strain) | CJ2004 (Mutant strain 1) | CJ2029 (Mutant strain 2) | CJ2049 (Mutant strain 3) |
| Glycine concentration (mg/L) | 110 | 135 | 192 | 138 |

As a result, as shown in Table 1, it was confirmed that the parent strain, *Corynebacterium glutamicum* ATCC13032 strain produced glycine at a concentration of 110 mg/L, but the mutant *Corynebacterium glutamicum* CJ2004 strain, CJ2029 strain and CJ2049 strain according to the present invention produced glycine at a concentration of 135 mg/L, 192 mg/L, 138 mg/L, respectively, and thus, the glycine productivity increased to about 22% to 74% compared to the parent strain.

The result means that the 3 kinds of the strains of the *Corynebacterium glutamicum* mutant strains according to the present invention can produce glycine with high efficiency and high yield.

### [Accession numbers]

Name of Depository Authority: Korean Culture Center of Microorganisms (KCCM) Accession number: KCCM13317P
Date of deposit: 20230106
Name of Depository Authority: Korean Culture Center of Microorganisms (KCCM) Accession number: KCCM13318P
Date of deposit: 20230106
Name of Depository Authority: Korean Culture Center of Microorganisms (KCCM) Accession number: KCCM13319P
Date of deposit: 20230106

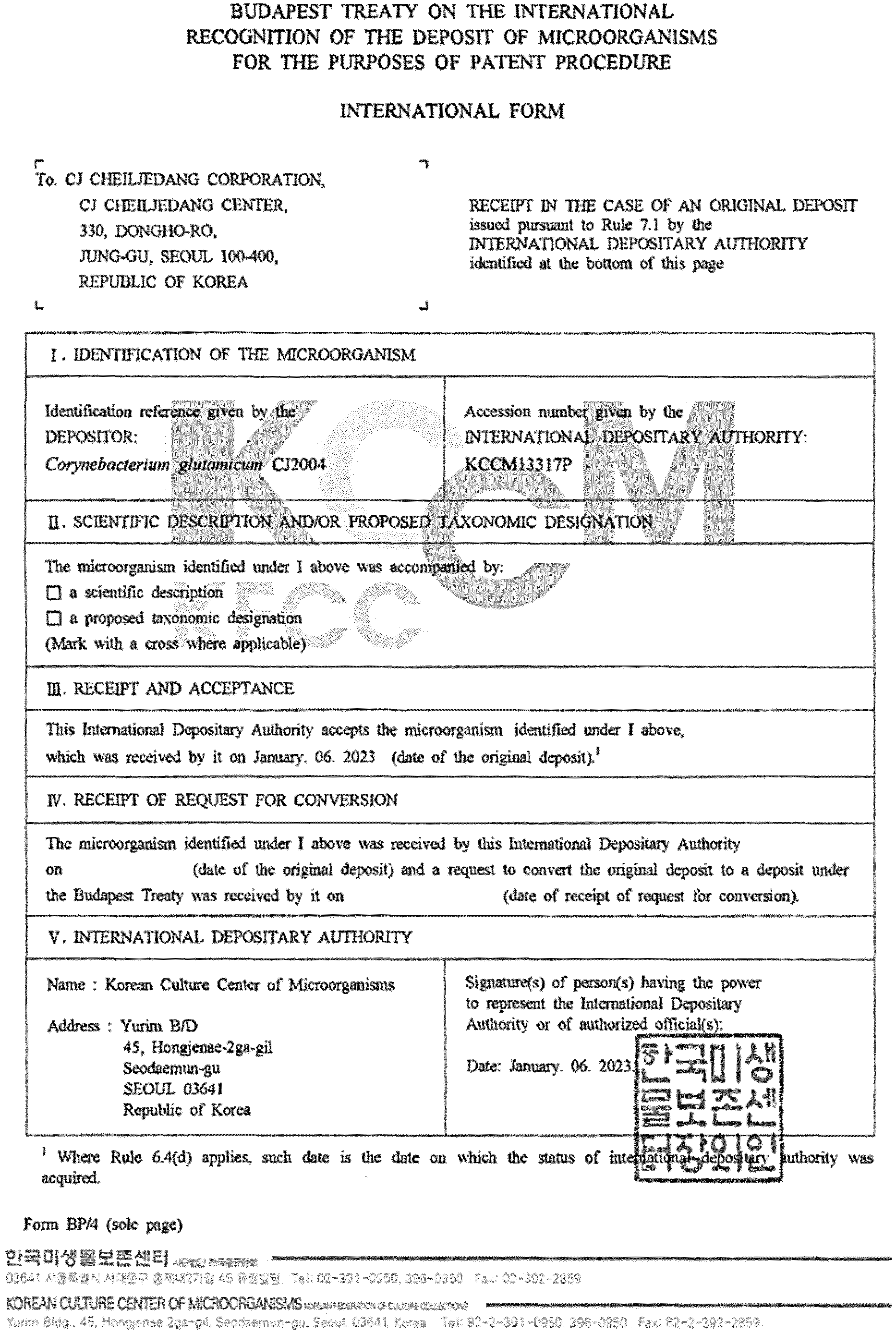
BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

To. CJ CHEILJEDANG CORPORATION,
CJ CHEILJEDANG CENTER,
330, DONGHO-RO,
JUNG-GU, SEOUL 100-400,
REPUBLIC OF KOREA

RECEIPT IN THE CASE OF AN ORIGINAL DEPOSIT
issued pursuant to Rule 7.1 by the
INTERNATIONAL DEPOSITARY AUTHORITY
identified at the bottom of this page

I. IDENTIFICATION OF THE MICROORGANISM
Identification reference given by the
DEPOSITOR:
Corynebacterium glutamicum CJ2004 | Accession number given by the
INTERNATIONAL DEPOSITARY AUTHORITY:
KCCM13317P
II. SCIENTIFIC DESCRIPTION AND/OR PROPOSED TAXONOMIC DESIGNATION
The microorganism identified under I above was accompanied by:
☐ a scientific description
☐ a proposed taxonomic designation
(Mark with a cross where applicable)
III. RECEIPT AND ACCEPTANCE
This International Depositary Authority accepts the microorganism identified under I above,
which was received by it on January. 06. 2023 (date of the original deposit).¹
IV. RECEIPT OF REQUEST FOR CONVERSION
The microorganism identified under I above was received by this International Depositary Authority
on (date of the original deposit) and a request to convert the original deposit to a deposit under
the Budapest Treaty was received by it on (date of receipt of request for conversion).
V. INTERNATIONAL DEPOSITARY AUTHORITY
Name : Korean Culture Center of Microorganisms

Address : Yurim B/D
45, Hongjenae-2ga-gil
Seodaemun-gu
SEOUL 03641
Republic of Korea | Signature(s) of person(s) having the power
to represent the International Depositary
Authority or of authorized official(s):

Date: January. 06. 2023.

¹ Where Rule 6.4(d) applies, such date is the date on which the status of international depositary authority was
acquired.

Form BP/4 (sole page)

한국미생물보존센터 [?]
03641 서울특별시 서대문구 홍제내2가길 45 유림빌딩 Tel: 02-391-0950, 396-0950 Fax: 02-392-2859
KOREAN CULTURE CENTER OF MICROORGANISMS KOREAN FEDERATION OF CULTURE COLLECTIONS
Yurim Bldg., 45, Hongjenae 2ga-gil, Seodaemun-gu, Seoul, 03641, Korea. Tel: 82-2-391-0950, 396-0950 Fax: 82-2-392-2859

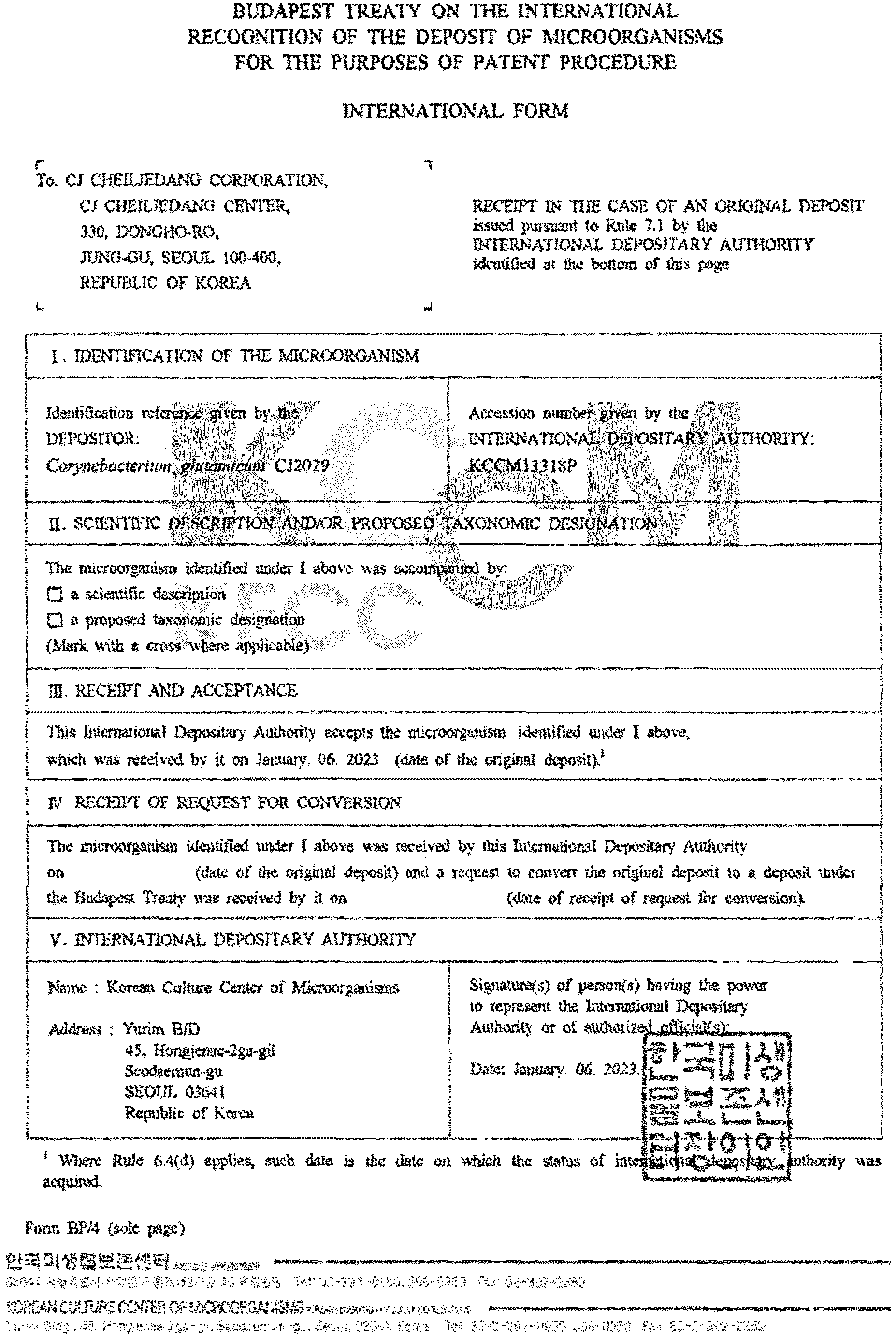
BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

To. CJ CHEILJEDANG CORPORATION,
CJ CHEILJEDANG CENTER,
330, DONGHO-RO,
JUNG-GU, SEOUL 100-400,
REPUBLIC OF KOREA

RECEIPT IN THE CASE OF AN ORIGINAL DEPOSIT
issued pursuant to Rule 7.1 by the
INTERNATIONAL DEPOSITARY AUTHORITY
identified at the bottom of this page

I. IDENTIFICATION OF THE MICROORGANISM
Identification reference given by the
DEPOSITOR:
Corynebacterium glutamicum CJ2029 | Accession number given by the
INTERNATIONAL DEPOSITARY AUTHORITY:
KCCM13318P
II. SCIENTIFIC DESCRIPTION AND/OR PROPOSED TAXONOMIC DESIGNATION
The microorganism identified under I above was accompanied by:
☐ a scientific description
☐ a proposed taxonomic designation
(Mark with a cross where applicable)
III. RECEIPT AND ACCEPTANCE
This International Depositary Authority accepts the microorganism identified under I above,
which was received by it on January. 06. 2023 (date of the original deposit).¹
IV. RECEIPT OF REQUEST FOR CONVERSION
The microorganism identified under I above was received by this International Depositary Authority
on (date of the original deposit) and a request to convert the original deposit to a deposit under
the Budapest Treaty was received by it on (date of receipt of request for conversion).
V. INTERNATIONAL DEPOSITARY AUTHORITY
Name : Korean Culture Center of Microorganisms

Address : Yurim B/D
45, Hongjenae-2ga-gil
Seodaemun-gu
SEOUL 03641
Republic of Korea | Signature(s) of person(s) having the power
to represent the International Depositary
Authority or of authorized official(s):

Date: January. 06. 2023.

¹ Where Rule 6.4(d) applies, such date is the date on which the status of international depositary authority was
acquired.

Form BP/4 (sole page)

한국미생물보존센터 [?]
03641 서울특별시 서대문구 홍제내2가길 45 유림빌딩 Tel: 02-391-0950, 396-0950 Fax: 02-392-2859
KOREAN CULTURE CENTER OF MICROORGANISMS KOREAN FEDERATION OF CULTURE COLLECTIONS
Yurim Bldg., 45, Hongjenae 2ga-gil, Seodaemun-gu, Seoul, 03641, Korea. Tel: 82-2-391-0950, 396-0950 Fax: 82-2-392-2859

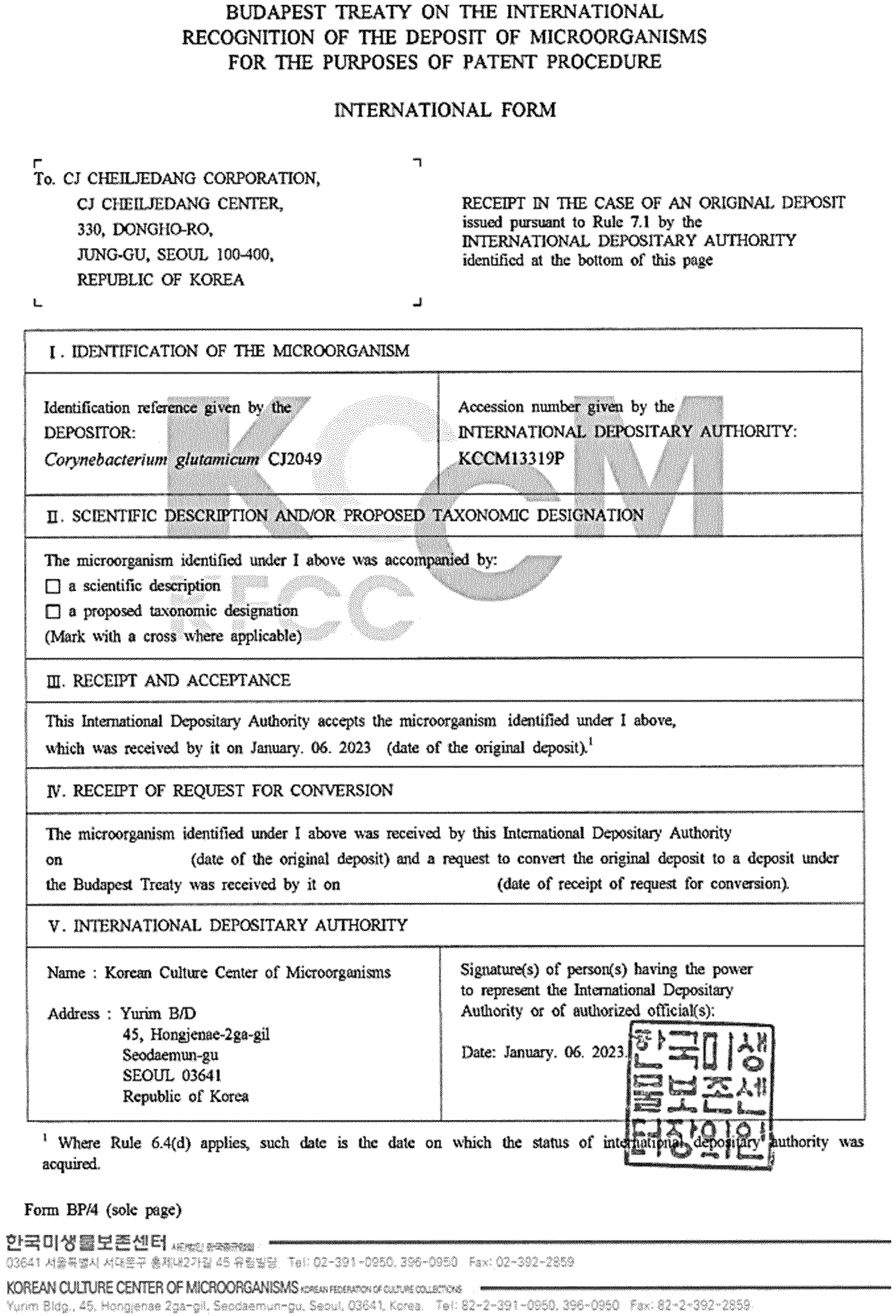
BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

To. CJ CHEILJEDANG CORPORATION,
CJ CHEILJEDANG CENTER,
330, DONGHO-RO,
JUNG-GU, SEOUL 100-400,
REPUBLIC OF KOREA

RECEIPT IN THE CASE OF AN ORIGINAL DEPOSIT
issued pursuant to Rule 7.1 by the
INTERNATIONAL DEPOSITARY AUTHORITY
identified at the bottom of this page

I. IDENTIFICATION OF THE MICROORGANISM
Identification reference given by the
DEPOSITOR:
Corynebacterium glutamicum CJ2049 | Accession number given by the
INTERNATIONAL DEPOSITARY AUTHORITY:
KCCM13319P
II. SCIENTIFIC DESCRIPTION AND/OR PROPOSED TAXONOMIC DESIGNATION
The microorganism identified under I above was accompanied by:
☐ a scientific description
☐ a proposed taxonomic designation
(Mark with a cross where applicable)
III. RECEIPT AND ACCEPTANCE
This International Depositary Authority accepts the microorganism identified under I above,
which was received by it on January. 06. 2023 (date of the original deposit).¹
IV. RECEIPT OF REQUEST FOR CONVERSION
The microorganism identified under I above was received by this International Depositary Authority
on (date of the original deposit) and a request to convert the original deposit to a deposit under
the Budapest Treaty was received by it on (date of receipt of request for conversion).
V. INTERNATIONAL DEPOSITARY AUTHORITY
Name : Korean Culture Center of Microorganisms

Address : Yurim B/D
45, Hongjenae-2ga-gil
Seodaemun-gu
SEOUL 03641
Republic of Korea | Signature(s) of person(s) having the power
to represent the International Depositary
Authority or of authorized official(s):

Date: January. 06. 2023.


¹ Where Rule 6.4(d) applies, such date is the date on which the status of international depositary authority was
acquired.

Form BP/4 (sole page)

한국미생물보존센터 [?]
03641 서울특별시 서대문구 홍제내2가길 45 유림빌딩 Tel: 02-391-0950, 396-0950 Fax: 02-392-2859
KOREAN CULTURE CENTER OF MICROORGANISMS KOREAN FEDERATION OF CULTURE COLLECTIONS
Yurim Bldg., 45, Hongjenae 2ga-gil, Seodaemun-gu, Seoul, 03641, Korea. Tel: 82-2-391-0950, 396-0950 Fax: 82-2-392-2859

## Claims

1. *A Corynebacterium* sp. mutant strain with improved glycine productivity compared to a parent strain, which is a mutant strain obtained by mutating a parent strain, *Corynebacterium glutamicum* ATCC13032 strain.

2. The *Corynebacterium* sp. mutant strain according to claim 1, wherein the *Corynebacterium* sp. mutant strain is *Corynebacterium glutamicum.*

3. The *Corynebacterium* sp. mutant strain according to claim 1, wherein the *Corynebacterium* sp. mutant strain is any one selected from the group consisting of *Corynebacterium glutamicum* CJ2004 strain (Accession number KCCM13317P), *Corynebacterium glutamicum* CJ2029 strain (Accession number KCCM13318P) and *Corynebacterium glutamicum* CJ2049 strain (Accession number KCCM13319P).

4. The *Corynebacterium* sp. mutant strain according to claim 1, wherein the mutant strain has 20% or more improved glycine productivity compared to a parent strain, *Corynebacterium glutamicum* ATCC13032.

5. A composition for producing glycine comprising the *Corynebacterium* sp. mutant strain of Claim 1.

6. The composition for producing glycine according to claim 5, wherein the *Corynebacterium* sp. mutant strain is any one selected from the group consisting of *Corynebacterium glutamicum* CJ2004 strain (Accession number KCCM13317P), *Corynebacterium glutamicum* CJ2029 strain (Accession number KCCM13318P) and *Corynebacterium glutamicum* CJ2049 strain (Accession number KCCM13319P).

7. A method for producing glycine, comprising culturing the *Corynebacterium* sp. mutant strain of claim 1 in a medium.

8. The method for producing glycine according to claim 7, wherein the *Corynebacterium* sp. mutant strain is any one selected from the group consisting of *Corynebacterium glutamicum* CJ2004 strain (Accession number KCCM13317P), *Corynebacterium glutamicum* CJ2029 strain (Accession number KCCM13318P) and *Corynebacterium glutamicum* CJ2049 strain (Accession number KCCM13319P).
